# NEUE EUROPÄISCHE PATENTSCHRIFT

(11) **EP 0 293 744 B2**
(45) Veröffentlichungstag und Bekanntmachung des Hinweises auf die Entscheidung über den Einspruch: **27.03.1996**
(45) Hinweis auf die Patenterteilung: 14.11.1990
(21) Anmeldenummer: 88108361.2
(22) Anmeldetag: 26.05.1988
(51) Int. Cl.: C07B 63/00

(54) **Verfahren zur Isolierung von Benzolmonosulfonsäuren aus wässriger Lösung oder Suspension**
Process for recovering benzolmonosulfonic acids from aqueous solutions or suspensions
Procédé pour l'obtention d'acides benzolmonsulfoniques à partir de solutions aqueuses ou de suspensions

(30) Priorität: 01.06.1987 DE 3718314
(43) Veröffentlichungstag der Anmeldung: 07.12.1988
(73) Patentinhaber: BASF Aktiengesellschaft, D-67063 Ludwigshafen (DE)
(72) Erfinder: Lamm, Gunther, Dr., D-6733 Hassloch (DE)

(56) Entgegenhaltungen:
- EP-A- 0 037 883
- DE-A- 1 668 048
- DE-A- 2 139 477
- DE-A- 2 205 300
- DE-A- 2 337 395
- JP-A- 1 042 180
- US-A- 3 957 859
- US-A- 4 175 092
- The Soviet Chemical Industry, 15:10, 1983, pp1183-1186

## Beschreibung

Die vorliegende Erfindung betrifft ein neues Verfahren zur Isolierung von Benzolmonosulfonsäuren aus wässriger Lösung oder Suspension durch Extraktion der wässrigen Lösung oder Suspension, in der die Benzolmonosulfonsäuren oder ihre Alkalisalze enthalten sind, mit einem mit Wasser nicht oder nur begrenzt mischbaren organischen Lösungsmittel aus der Klasse der Alkohole, Ketone oder Carbonsäureester, Abtrennung der organischen Phase und Entfernung des organischen Lösungsmittels.

Aromatische Sulfonsäuren besitzen im allgemeinen eine gute Wesserlöslichkeit und ihre Isolierung aus wässriger Lösung oder Suspension bereitet daher häufig Schwierigkeiten. Dieses Problem tritt insbesondere bei der Synthese von aromatischen Sulfonsäuren auf. Üblicherweise werden diese Sulfonsäuren durch Sulfonierung der entsprechenden aromatischen Verbindungen mit Schwefelsäure und/oder Oleum gewonnen, wobei nach beendeter Reaktion das Reaktionsgemisch auf Eis ausgetragen und gegebenenfalls mit Natronlauge abgestumpft wird. Nach herkömmlichen Methoden, d. h. durch Bildung der jeweiligen Natriumsulfonate oder auch von Betainen, gelingt es im allgemeinen jedoch nicht, die gewünschten aromatischen Sulfonsäuren quantitativ aus der wässrigen Lösung zu entfernen, wodurch einerseits die Ausbeute an Wertprodukt herabgesetzt und andererseits das Abwasser durch diese Sulfonsäuren kontaminiert wird.

Aus der DE-A-2 139 477 ist die Abtrennung von Paraffinsulfonsäuren aus einem wäßrigen Sulfonierungsgemisch bekannt, wobei Alkohole, die mindestens 5 Kohlenstoffatome im Molekül aufweisen, als Extraktionsmittel dienen.

In diesem Fall weisen die zu isolierenden Komponenten aufgrund ihrer langen hydrophoben Alkylketten von vorneherein eine gute Verträglichkeit gegenüber dem verwendeten Extraktionsmittel auf. Aufgrund ihrer anderen Molekülstruktur konnte aber von aromatischen Sulfonsäure, deren Polarität bekannt ist, und insbesondere von solchen aromatischen Sulfonsäuren, die zusätzlich durch polare Gruppen substituiert sind, nicht erwartet werden, daß sie unter diesen Bedingungen extrahiert werden könnten.

Es war zu erwarten, daß dazu stärker polare Lösungsmittel notwendig sind. So wird in der FR-A-2 532 318 beschrieben, sulfonierte Triarylphosphine mittels Estern der Phosphorsäure, der Phosphonsäure oder der Phosphinsäure oder mittels Phosphinoxiden aus der wässrigen Phase zu extrahieren. Die dort genannten Extraktionsmittel sind jedoch stark toxisch und kommen für die industrielle Anwendung nicht in Betracht.

In der JP-A-10 421/1980 wird ein Verfahren zu Extraktion von Toluolmonosulfonsäuren beschrieben, mit Hilfe von ausgewählten Alkoholen, Ketonen, Fettsäureestern, aliphatischen Carbonsäuren oder cyclischen Ethern. Die Gegenwart von Ammoniumsulfat ist zwingend vorgeschrieben.

Aufgabe der vorliegenden Erfindung war es, ein Verfahren bereitzustellen, bei dem die voranstehend genannten Mängel auf einfache Weise behoben werden und das ohne großen zusätzlichen apparativen und sicherheitstechnischen Aufwand betrieben werden kann.

Es wurde nun gefunden, daß die Isolierung von Benzolmonosulfonsäuren, die ein- oder mehrfach substituiert sein können, in Form der freien Säure oder in Form ihrer Alkalisalze aus wäßriger Lösung oder Suspension vorteilhaft gelingt, wenn man die wäßrige Lösung oder Suspension, in der die Benzolmonosulfonsäuren oder ihre Alkalizalze enthalten sind, mit einem mit Wasser nicht oder nur begrenzt mischbaren organischen Lösungsmittel aus der Klasse der Alkohole, Ketone oder Carbonsäureester extrahiert, indem man die wäßrige Lösung oder Suspension mit dem organischen Lösungsmittel rührt, die organische Phase abtrennt, gegebenenfalls die darin enthaltenen freien Benzolmonosulfonsäuren in ihre Alkalisalze überführt und dann das organische Lösungsmittel entfernt.

Geeignete Substituenten sind beispielsweise Halogen, Hydroxy, C₁-C₄-Alkyl, C₁-C₄-Alkoxy, Nitro, Amino, C₁-C₄-Mono- oder Dialkylamino, Pyrrolidino, Piperidino, Morpholino, Piperazino, (N-C₁-C₄-Alkyl)piperazino, C₁-C₄-Dialkylaminomethyl, 2-(C₁-C₄-Dialkylamino)ethyl, (N-C₁-C₄-Alkyl-N-phenylamino)methyl, 2-(N-C₁-C₄-Alkyl-N-phenylamino)ethyl (in den beiden letztgenannten Fällen kann die an der Aminogruppe befindliche Phenylgruppe weiterhin beispielsweise durch Halogen, C₁-C₄-Alkyl oder C₁-C₄-Alkoxy substituiert sein), 5-(C₁-C₄-Alkyl)pyrazol-3-on-1-yl, 3-(C₁-C₄-Alkyl)pyrazol-5-on-1-yl oder 2-[3- oder 5-Cyano- oder -carbamoyl-4-(C₁-C₄-alkyl)-6-halogenpyridin-1-ylamino]ethyl.

Beispielhaft seien die folgenden Sulfonsäuren genannt: 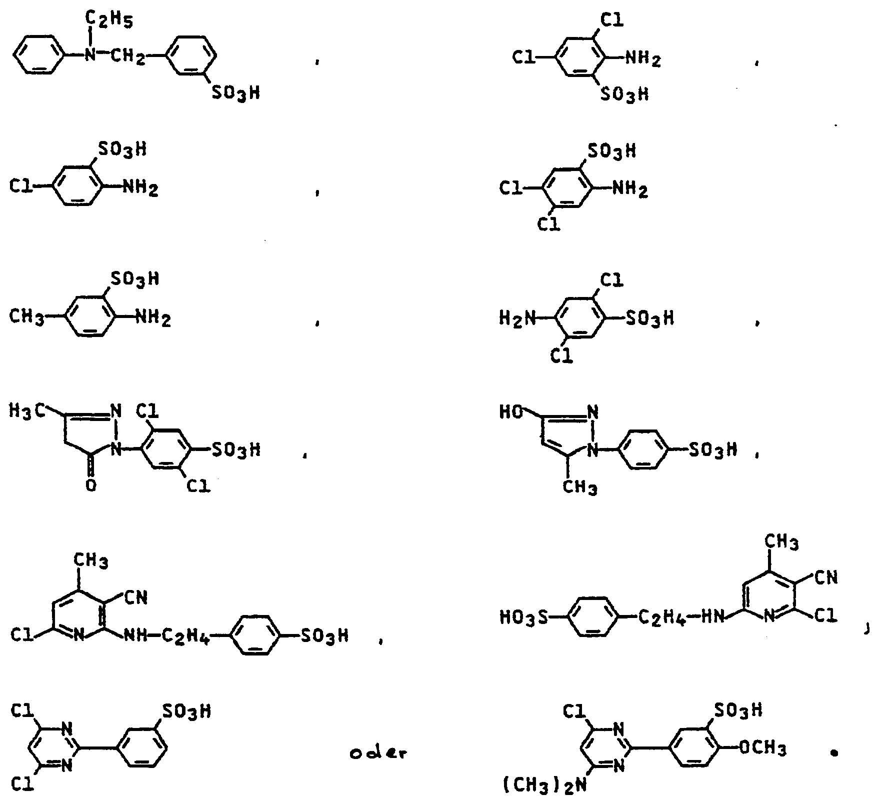
Unter den Alkalisalzen sind die Lithium-, Natrium- oder Kaliumsalze zu verstehen, wobei die Natriumsalze bevorzugt sind.

Als Extraktionsmittel für das erfindungsgemäße Verfahren dienen organische Lösungsmittel aus der Klasse der Alkohole, Ketone oder Carbonsäureester, die mit Wasser nicht oder nur begrenzt mischbar sind.

Beispielhaft seien die folgenden Lösungsmittel genannt:
Alkohole: C₄-C₁₀-Alkanole oder C₅-C₇-Cycloalkanole, z. B. Butanol, Isobutanol, sec-Butanol, Pentanol, Isopentanol, sec-Pentanol, Hexanol, 2-Ethylhexanol, Cyclopentanol, Cyclohexanol, 4-Methylcyclohexanol oder Cycloheptanol
Ketone: C₄-C₈-Alkanone oder C₅-C₇-Cycloalkanone, z. B. Methylethylketon, Diethylketon, Diisopropylketon, Di-n-propylketon, Methylisopentylketon, Cyclopentanon, Cyclohexanon oder Cycloheptanon.
Carbonsäureester: C₁-C₈ Alkylester der Essigsäure, Methoxyessigsäure oder Propionsäure oder cyclische Ester, z. B. Essigsäureethylester, Essigsäurebutylester, Methoxyessigsäureethylester, Propionsäureethylester oder γ-Butyrolacton.

Das erfindungsgemäße Verfahren wird vorzugsweise mit Alkoholen, C₄-C₈-Alkanonen oder Carbonsäureestern, die insgesamt bis zu 10 Kohlenstoffatome aufweisen, als Extraktionsmittel durchgeführt. Insbesondere die Verwendung von C₄-C₈-Alkanolen ist bevorzugt. Besonders hervorzuheben sind dabei Butanol, Pentanol und insbesondere Isobutanol.

Zweckmäßig führt man das erfindungsgemäße Verfahren so durch, daß man die wässrige Lösung oder Suspension, die die Benzolmonosulfonsäure oder ihr Alkalisalz enthält, mit dem organischen Lösungsmittel versetzt und bei einer Temperatur von 0 bis 100°C, vorzugsweise 15 bis 90°C, über einen Zeitraum von 5 bis 60 Minuten, vorzugsweise 5 bis 30 Minuten, rührt.

Nach Beendigung der Rührphase erfolgt eine Phasentrennung. Die organische Phase (in der Regel leichter) wird abgetrennt und auf an sich bekannte Weise, z. B. durch Destillation, gegebenenfalls unter vermindertem Druck, oder durch Trocknen im Schaufeltrockner vom organischen Lösungsmittel befreit. Das Zielprodukt fällt dabei in fester Form, in wäßriger Lösung oder als Suspension an. Das organische Lösungsmittel kann zurückgewonnen und erneut zur Extraktion verwendet werden.

Das Gewichtsverhältnis von Sulfonsäure (bzw. deren Alkalisalz) zu organischem Lösungsmittel beträgt beim erfindungsgemäßen Verfahren z.B. ca. 1:1 bis 1:5, vorzugsweise 1:1 bis 1:3.

Um Verluste des organischen Extraktionsmittels zu vermeiden oder gering zu halten, ist es bei Verwendung von begrenzt wasserlöslichen Extraktionsmitteln, z. B. Isobutanol, vorteilhaft, die Extraktion mit einer wässrigen Lösung oder Suspension vorzunehmen, die gesättigt an Natriumchlorid oder Natriumsulfat ist.

Vorteilhaft können wässrige Reaktionsgemische, wie sie bei der Aufarbeitung von Sulfonierungsreaktionen an Benzolen anfallen, mittels des erfindungsgemäßen Verfahrens extrahiert werden.

Für den Fall, daß die Sulfonsäuren in Form ihrer Alkalisalze, insbesondere als Natriumsalz, leichter extrahierbar sind als die freien Säuren, kann man die Bildung des Salzes in wässriger Lösung vornehmen und dieses anschließend der Extraktion unterwerfen. Es ist aber auch möglich, die Salzbildung nach Extraktion der freien Sulfonsäuren aus dem wässrigen Milieu direkt in der organischen Phase durch Zugabe der entsprechenden Alkalihydroxide oder Alkalicarbonate vorzunehmen.

In manchen Fällen, insbesondere bei einigen Natriumsulfonaten oder bei freien Sulfonsäuren, die in der Betainform vorliegen, kann es zur Ausbildung einer Suspension in der organischen Phase kommen. Dies ist aber nicht weiter hinderlich, da in diesem Falle die organische Phase als Suspension von der wässrigen Phase abgetrennt und in üblicher Weise aufgearbeitet wird.

Im allgemeinen ist es ausreichend, eine einmalige Extraktion der wässrigen Phase durchzuführen, um die Benzolmonosulfonsäuren in einer Ausbeute von 95% und darüber aus der wässrigen Phase zu isolieren. In manchen Fällen kann es jedoch auch von Vorteil sein, eine zweimalige Extraktion vorzunehmen.

Das erfindungsgemäße Verfahren, das sowohl in kontinuierlicher, als auch in diskontinuierlicher Arbeitsweise durchgeführt werden kann, eignet sich nicht nur zur Extraktion von Benzolmonosulfonsäuren aus Reaktionsgemischen (bei deren Aufarbeitung), sondern ist generell für die Isolierung von Benzolmonosulfonsäuren oder deren Alkalisalze aus wässriger Lösung oder Suspension geeignet.

Das neue Verfahren erlaubt es, Benzolmonosulfonsäuren oder deren Alkalisalze auf einfache Weise zu extrahieren, wobei die Konzentration der Benzolmonosulfonsäuren und insbesondere ihrer Alkalisalze im erfindungsgemäß zu verwendenden Extraktionsmittel erstaunlich hoch ist (bis zu ca. 50 Gew.%).

Die mittels des erfindungsgemäßen Verfahrens isolierbaren Benzolmonosulfonsäuren sind z. B. wertvolle Zwischenprodukte für die Synthese von Farbstoffen.

Die folgenden Beispiele sollen die Erfindung näher erläutern. Für den Fall, daß in den nachfolgend aufgeführten Formeln folgendes Strukturelement vorkommt 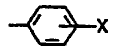
bedeutet dies, daß es sich um Isomerengemische handelt, bei denen dasjenige Isomere, das durch die Position von X formelmäßig charakterisiert ist, als Hauptkomponente vorliegt.

### Beispiel 1

Ein Gemisch aus 190 g der Verbindungen A¹-H und 81,5 g der Verbindung A²-H 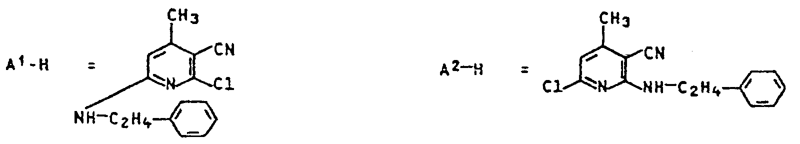
wurde unter Solekühlung bei 10 - 18°C in eine Mischung aus 300 g 96 gew.%iger Schwefelsäure und 500 g 24 gew.%igem Oleum eingetragen. Man rührte 8 Stunden bei 15 - 17°C und gab das Reaktionsgemisch dann auf 1600 g Eis und 300 g 50 gew.%ige Natronlauge. Danach versetzte man das entstandene Gemisch mit 500 ml Isobutanol, rührte 15 Minuten bei 35-50°C und ließ dann von der wäßrigen Phase absitzen. Nach 10 Minuten trennte man die Isobutanol-Phase ab und neutralisierte sie mit 94 g Natronlauge (50 gew.%ig) und 3 g Natriumcarbonat. Nach Einengen im Schaufeltrockner erhielt man 373,5 g eines kristallinen Pulvers das neben ca. 14,5 g Natriumsulfat insgesamt 359 g der Verbindungen A¹-SO₃Na und A²-SO₃Na enthielt, wobei das Mischungsverhältnis gegebenüber den Einsatzstoffen unverändert war.

Die Sulfierungsprodukte haben die Formel 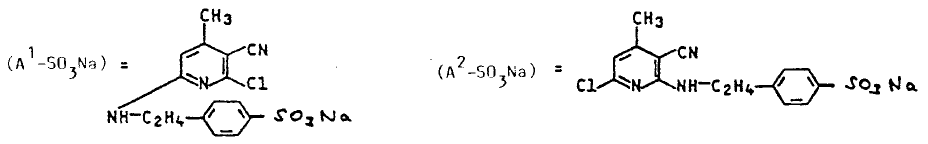
Das zurückgewonnene Isobutanol wurde erneut zur Abtrennung von A¹-SO₃H und A²-SO₃H eingesetzt. Durch eine zweite Extraktion ließen sich die geringen Restanteile von A¹-SO₃H und A²-SO₃H aus der wäßrigen, schwefelsauren Phase quantitativ entfernen.

### Bespiel 2

211 g (N-Ethyl-N-phenyl)benzylamin wurden bei einer Temperatur unterhalb von 20°C in 500 g Oleum (24 gew.%ig) eingerührt. Dann steigerte man die Temperatur langsam auf ca 70°C, bis die Reaktion beendet war. Das Gemisch wurde auf 1500 g Eis und 300 g 50 gew.%ige Natronlauge gegeben und mit 1000 ml Isobutanol überschichtet. Dann gab man unter Rühren soviel 50 gew.%ige Natronlauge hinzu, bis der pH-Wert des Gemisches im Bereich von 7 - 11 lag. Man rührte dann ca. 10 Minuten bei 40 - 70°C und trennte die wäßrige Phase danach ab. Die organische Phase wurde im Schaufeltrockner getrocknet Man erhielt 306,5 g eines farblosen Pulvers der Formel 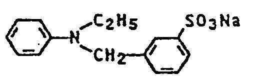
das zusätzlich noch ca. 10 g Kristallwasser und Spuren Natriumsulfat enthielt.

Als man das Reaktionsgemisch der Sulfonierungsreaktion auf 2000 g Eis (anstelle von 1500 g Eis) gab, erhielt man das Produkt auf ähnlich günstige Weise.

Man konnte die Isobutanol-Lösung von (N-Ethyl-N-phenyl)benzylaminsulfonsäure-Na-Salz aber auch wie folgt aufarbeiten:
a) Man destillierte Isobutanol unter atmosphärischem Druck bis zu einer Temperatur von ca. 97°C ab, versetzte den öligen Rückstand mit 150 ml Wasser, destillierte weitere 100 ml Wasser/Isobutanol-Gemisch ab und enthielt eine mit Wasser verdünnbare Lösung des Wertproduktes. Diese Lösung konnte direkt zu Kupplungsreaktionen eingesetzt werden.
b) Andererseits konnte man die unter a) erhaltene konzentrierte isobutanolfreie Lösung auch mit 200 ml Wasser versetzen, auf 40°C abkühlen und mit Schwefelsäure auf einen pH-Wert im Bereich von ca. 0,5 -1 ansäuern, so daß das Wertprodukt aus konzentrierter Lösung (Gesamtvolumen ca. 660 ml) in Form des Betains grobkristallin ausfiel. Es wurde dann wie üblich aubgesaugt und getrocknet.

Die Ausbeute betrug 94 % der Theorie. Durch Wiederverwendung des Filtrates (als Vorlage zur Fällung nach der Sulfierung) konnte die Ausbeute auf über 98 % gesteigert werden.

### Beispiel 3

162 g 2,4-Dichloranilin wurden bei Raumtemperatur in 200 g 5 gew.%igem Oleum gelöst. Dann erhitzte man das Gemisch auf 100°C und tropfte innerhalb von 1 Stunde 180 ml Chlorsulfonsäure bei 100 - 125°C hinzu. Anschließend steigerte man die Reaktionstemperatur auf 135 - 140°C und rührte 4,5 Stunden nach. Danach kühlte man auf 80°C ab und gab das Reaktionsgemisch auf 1500 g Eis und 300 g 50 gew.%iger Natronlauge. Das resultierende Gemisch wurde analog Beispiel 1 zweimal mit je 800 ml Isobutanol extrahiert. Die organischen Phasen wurden vereinigt und mit wäßriger Natronlauge neutralisiert. Nach dem Abdampfen von Isobutanol unterwarf man den Rückstand einer Wasserdampfdestillation, um nicht umgesetztes Ausgangsprodukt abzutrennen. Danach wurde der Rückstand auf Raumtemperatur abgekühlt und das ausgefallene Produkt der Formel 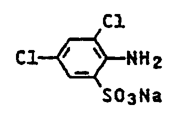
abgesaugt und getrocknet. Man erhielt 251 g eines farblosen Pulvers.

### Beispiel 4

1 Mol (N-Ethyl-N-phenyl)benzylamin-3-sulfonsäure wurde analog Beispiel 2 hergestellt. Anstelle von Isobutanol verwendete man jedoch jeweils n-Butanol, Methylethylketon, Diethylketon, Essigsäurebutylester oder Cyclohexanon als Extraktionsmittel. Dabei ließ sich das Natriumsalz der (N-Ethyl-N-phenyl)benzylamin-3-sulfonsäure in ähnlicher Ausbeute extrahieren und nach Entfernen des jeweiligen Lösungsmittels in fester Form isolieren.

Die in den folgenden Tabellen 1 - 3 aufgeführten Produkte lassen sich analog zu den in den voranstehenden Beispielen beschriebenen Arbeitsweisen isolieren. Bei den Beispielen Nr. 6, 7, 12, 13 und 27 erfolgte die Isolierung allerdings nicht im Rahmen einer Aufarbeitung des Reaktionsgemischs, sondern aus einer eigens bereiteten wäßrigen Lösung bzw. Suspension, die mit Schwefelsäure zusätzlich angesäuert wurde.

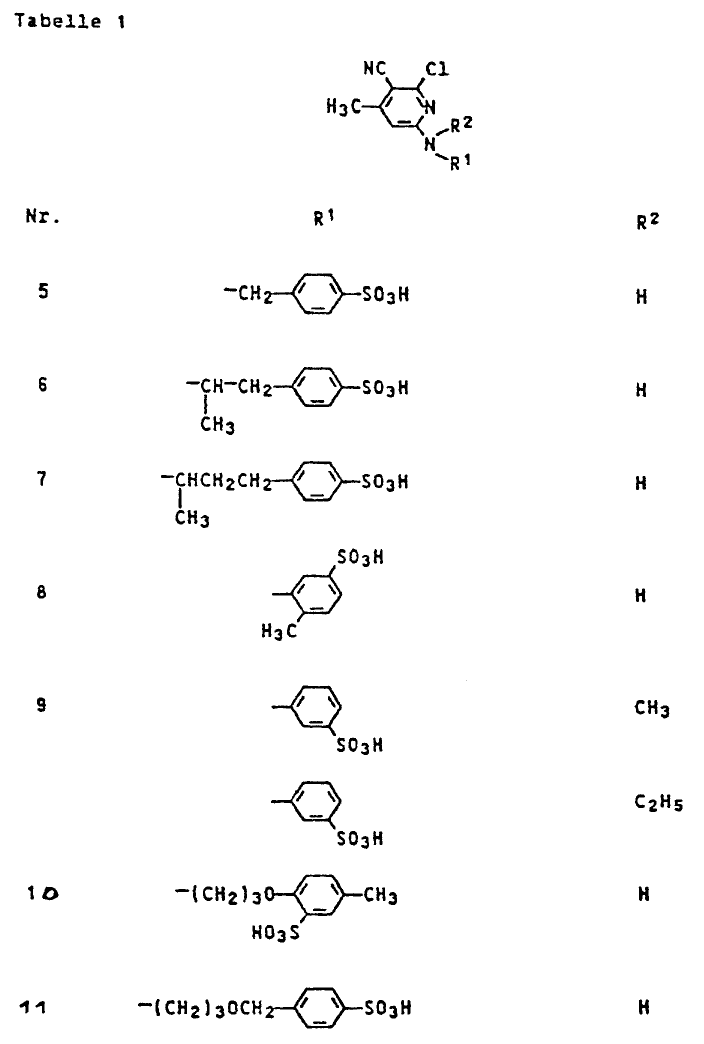

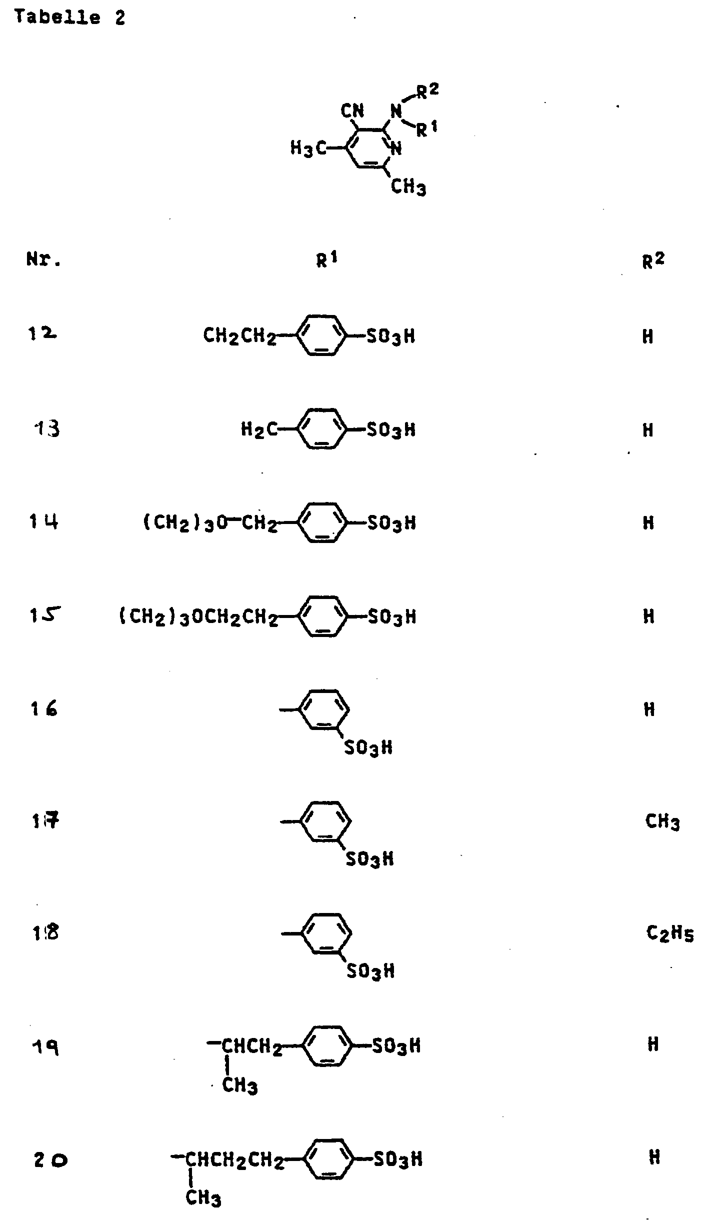

Anstelle der Cyanopyridine in den Tabellen 1 und 2 können jeweils auch die entsprechenden Carbamoyl-Verbindungen eingesetzt werden.

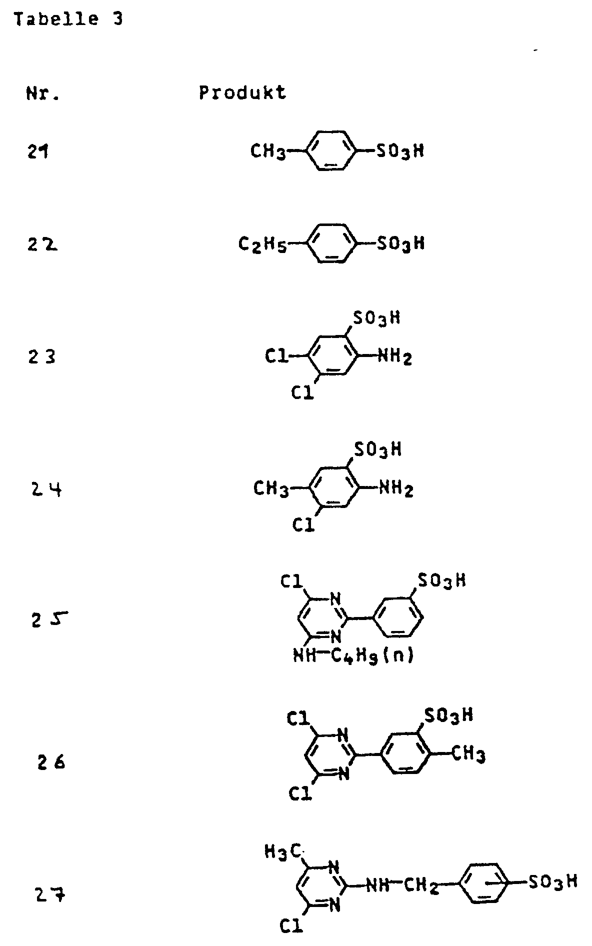

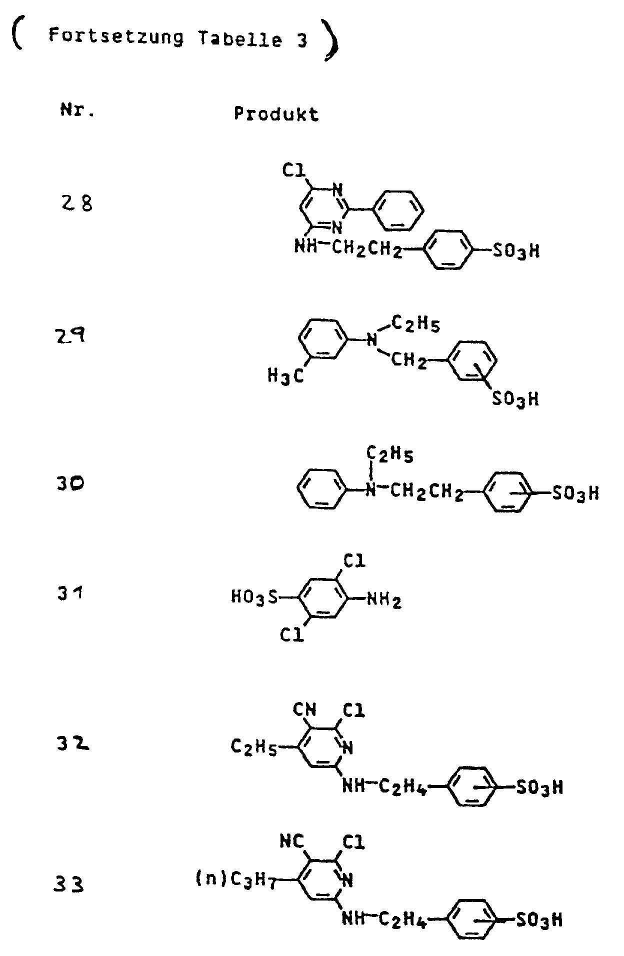

## Patentansprüche

1. Verfahren zur Isolierung von Benzolmonosulfonsäuren, die ein- oder mehrfach substituiert sein können, in Form der freien Säure oder in Form ihrer Alkalisalze aus wäßriger Lösung oder Suspension, dadurch gekennzeichnet, daß man die wäßrige Lösung oder Suspension, in der die Benzolmonosulfonsäuren oder ihre Alkalisalze enthalten sind, mit einem mit Wasser nicht oder nur begrenzt mischbaren organischen Lösungsmittel aus der Klasse der Alkohole, Ketone oder Carbonsäureester extrahiert, indem man die wäßrige Lösung oder Suspension mit dem organischen Lösungsmittel rührt, die organische Phase abtrennt, gegebenenfalls die darin enthaltenen freien Benzolmonosulfonsäuren in ihre Alkalisalze überführt und dann das organische Lösungsmittel entfernt.

2. Verfahren gemäß Anspruch 1, dadurch gekennzeichnet, daß man die Extraktion mit einem Alkohol als Lösungsmittel durchführt.

3. Verfahren gemäß Anspruch 1, dadurch gekennzeichnet, daß man die Extraktion mit C₄-C₈-Alkanol als Lösungsmittel durchführt.

## Claims

1. A process for isolating benzenemonosulfonic acids, which may be monosubstituted or polysubstituted, in the form of the free acid or in the form of their alkali metal salts from an aqueous solution or suspension, which comprises extracting the aqueous solution or suspension containing the benzenemonosulfonic acids or the alkali metal salts thereof with an organic solvent from the class of the alcohols, ketones or carboxylic esters which has only limited miscibility with water, if any, by stirring the aqueous solution or suspension with the organic solvent, separating off the organic phase, if necessary converting the free benzenemonosulfonic acids present therein into their alkali metal salts, and then removing the organic solvent.

2. A process as claimed in claim 1, wherein the extraction is carried out with an alcohol as solvent.

3. A process as claimed in claim 1, wherein the extraction is carried out with a C₄-C₈-alkanol as solvent.

## Revendications

1. Procédé d'isolement d'acides benzènemonosulfoniques, qui peuvent être substitués une ou plusieurs fois, sous forme des acides libres ou sous forme de leurs sels alcalins, à partir de solutions ou de suspensions aqueuses, caractérisé en ce qu'on extrait les solutions ou suspensions aqueuses dans lesquelles sont contenus les acides benzènemonosulfoniques ou leurs sels alcalins, avec un solvant organique non miscible ou de miscibilité limitée avec l'eau de la classe des alcools, des cétones ou des esters carboxyliques avec agitation de la solution ou suspension aqueuse avec le solvant organique, on sépare la phase organique, on transforme éventuellement les acides benzènemonosulfoniques libres qui y sont contenus en leurs sels alcalins puis on chasse le solvant organique.

2. Procédé selon la revendication 1, caractérisé en ce qu'on effectue l'extraction avec un alcool comme solvant.

3. Procédé selon la revendication 1, caractérisé en ce qu'on effectue l'extraction avec un alcanol en C₄-C₈ comme solvant.
